# EUROPEAN PATENT APPLICATION

(11) **EP 2 468 866 A1**
(43) Date of publication of application: **27.06.2012**
(21) Application number: 10196273.6
(22) Date of filing: 21.12.2010
(51) Int. Cl.: C12N 15/117, A61P 37/02, A61P 31/00, A61P 35/00

(54) **Biologically active oligonucleotides capable of modulating the immune system**

(71) Applicant: Index Pharmaceuticals AB, 171 77 Stockholm (SE)
(72) Inventor: Kouznetzov, Nikolai, 176 72 Järfälla (SE); Zargari, Arezou, 170 74 Solna (SE); Admyre, Charlotte, 116 35 Stockholm (SE); Von Stein, Oliver, 194 34 Upplands Väsby (SE); Von Stein, Petra, 194 34 Upplands Väsby (SE)
(74) Representative: Lundquist, Tomas

(57) **Abstract**

The present invention relates to methods for identifying oligonucleotides capable of modulating the immune system in a mammalian subject, comprising analysis of which tertiary structural form said oligonucleotide adopts, in aqueous solution. Further, the invention provides oligonucleotides identifiable by the methods of the invention and to their use in methods of treating diseases, such as inflammatory diseases, infectious diseases, neurodegenerative diseases, autoimmune diseases and cancer.

SEQUENCE LISTING

<110>  Index Pharmaceuticals AB

<120>  BIOLOGICALLY ACTIVE OLIGONUCLEOTIDES CAPABLE OF MODULATING THE
       IMMUNE SYSTEM I

<130>  21047727

<160>  94

<170>  PatentIn version 3.5

<210>  1
<211>  6
<212>  DNA
<213>  Artificial Sequence

<220>
<223>  IDX-0400

<400>  1
tggggt                                                                  6


<210>  2
<211>  11
<212>  DNA
<213>  Artificial Sequence

<220>
<223>  IDX-0405

<400>  2
gcggtttgcg g                                                           11


<210>  3
<211>  10
<212>  DNA
<213>  Artificial Sequence

<220>
<223>  IDX-0415

<400>  3
gggttttggg                                                             10


<210>  4
<211>  12
<212>  DNA
<213>  Artificial Sequence

<220>
<223>  IDX-0420

<400>  4
ggggttttgg gg                                                          12


<210>  5
<211>  7
<212>  DNA
<213>  Artificial Sequence

<220>
<223>  IDX-0430

<400>  5
gcatgct                                                                 7


<210>  6
<211>  20
<212>  DNA
<213>  Artificial Sequence

<220>
<223>  IDX-0435

<400>  6
ggttttggtt ttggttttgg                                                  20


<210>  7
<211>  18
<212>  DNA
<213>  Artificial Sequence

<220>
<223>  IDX-9011


<220>
<221>  phosphorothioate modification
<222>  (1)..(18)

<400>  7
tcacgaccgt caaactcc                                                    18


<210>  8
<211>  15
<212>  DNA
<213>  Artificial Sequence

<220>
<223>  IDX-0410

<400>  8
ggttggtgtg gttgg                                                       15


<210>  9
<211>  22
<212>  DNA
<213>  Artificial Sequence

<220>
<223>  IDX-0470

<400>  9
tcgtcgttct gccatcgtcg tt                                               22


<210>  10
<211>  22
<212>  DNA
<213>  Artificial Sequence

<220>
<223>  IDX-0475


<220>
<221>  phosphorothioate modification
<222>  (1)..(22)

<400>  10
ttgttgttct gccatcgtcg tt                                                22


<210>  11
<211>  22
<212>  DNA
<213>  Artificial Sequence

<220>
<223>  IDX-0480


<220>
<221>  phosphorothioate modification
<222>  (1)..(22)

<400>  11
tgctgcttct gccatgctgc tt                                                22


<210>  12
<211>  22
<212>  DNA
<213>  Artificial Sequence

<220>
<223>  IDX-0485


<220>
<221>  phosphorothioate modification
<222>  (1)..(22)

<400>  12
tcgtcgttct gccatcgtcg tt                                                22


<210>  13
<211>  27
<212>  DNA
<213>  Artificial Sequence

<220>
<223>  IDX-9013


<220>
<221>  phosphorothioate modification
<222>  (1)..(27)

<400>  13
tcgttcgtct tgttcgtttg ttcgtgg                                           27


<210>  14
<211>  28
<212>  DNA
<213>  Artificial Sequence

<220>
<223>  IDX-9014


<220>
<221>  phosphorothioate modification
<222>  (1)..(28)

<400>  14
tcgttcgtct tttcgttttc gtcggcgc                                          28


<210>  15
<211>  23
<212>  DNA
<213>  Artificial Sequence

<220>
<223>  IDX-9017


<220>
<221>  phosphorothioate modification
<222>  (1)..(23)

<400>  15
tccgcgttcg ttgttcgtcg cgg                                               23


<210>  16
<211>  21
<212>  DNA
<213>  Artificial Sequence

<220>
<223>  IDX-9028


<220>
<221>  phosphorothioate modification
<222>  (1)..(21)

<400>  16
cggcgcgccg ttcgtcgatg g                                                 21


<210>  17
<211>  19
<212>  DNA
<213>  Artificial Sequence

<220>
<223>  IDX-9029


<220>
<221>  phosphorothioate modification
<222>  (1)..(19)

<400>  17
cggcgccgtt cgtcgatgg                                                    19


<210>  18
<211>  26
<212>  DNA
<213>  Artificial Sequence

<220>
<223>  IDX-9069


<220>
<221>  phosphorothioate modification
<222>  (1)..(26)

<400>  18

tcgtctgctt gttcgtcttg ttcgtc                                          26

<210>  19
<211>  30
<212>  DNA
<213>  Artificial Sequence

<220>
<223>  IDX-9070

<220>
<221>  phosphorothioate modifiaction
<222>  (1)..(30)

<400>  19
tcgttcgtct gcttgttcgt cttgttcgtc                                      30

<210>  20
<211>  24
<212>  DNA
<213>  Artificial Sequence

<220>
<223>  IDX-9072

<220>
<221>  phosphorothioate modification
<222>  (1)..(24)

<400>  20
tcgttcgtct tgttcgtcgt ctgc                                            24

<210>  21
<211>  21
<212>  DNA
<213>  Artificial Sequence

<220>
<223>  IDX-9073

<220>
<221>  phosphorothioate modification
<222>  (1)..(21)

<400>  21
tcgttcgtct tgttcgtctg c                                               21

<210>  22
<211>  24
<212>  DNA
<213>  Artificial Sequence

<220>
<223>  IDX-9091

<220>
<221>  phosphorothioate modification
<222>  (1)..(24)

<400>  22
ttttcgtctg ctttcgtttc gttt                                            24

<210>  23
<211>  23
<212>  DNA
<213>  Artificial Sequence

<220>
<223>  IDX-9100


<220>
<221>  phosphorothioate modification
<222>  (1)..(23)

<400>  23
tgccattcgt cgttctcgtc gtt                                              23


<210>  24
<211>  21
<212>  DNA
<213>  Artificial Sequence

<220>
<223>  IDX-9138


<220>
<221>  phosphorothioate modification
<222>  (1)..(21)

<400>  24
tcgtcgttct gccatcgtcg t                                                21


<210>  25
<211>  17
<212>  DNA
<213>  Artificial Sequence

<220>
<223>  IDX-9139


<220>
<221>  phosphorothioate modification
<222>  (1)..(17)

<400>  25
tcgtcgttct cgtcgtt                                                     17


<210>  26
<211>  16
<212>  DNA
<213>  Artificial Sequence

<220>
<223>  IDX-9140


<220>
<221>  phosphorothioate modification
<222>  (1)..(16)

<400>  26
tcgttctgct gatcgt                                                      16

<210>  27
<211>  19
<212>  DNA
<213>  Artificial Sequence

<220>
<223>  IDX-9141


<220>
<221>  phosphorothioate modification
<222>  (1)..(19)

<400>  27
tcgtcgttct gtcgtcgtt                                                    19


<210>  28
<211>  18
<212>  DNA
<213>  Artificial Sequence

<220>
<223>  IDX-9142


<220>
<221>  phosphorothioate modification
<222>  (1)..(18)

<400>  28
tcgtcgttcg tcgtcgtt                                                     18


<210>  29
<211>  18
<212>  DNA
<213>  Artificial Sequence

<220>
<223>  IDX-9143


<220>
<221>  phosphorothioate modification
<222>  (1)..(18)

<400>  29
tcgtcgttgc tcgtcgtt                                                     18


<210>  30
<211>  16
<212>  DNA
<213>  Artificial Sequence

<220>
<223>  IDX-9144


<220>
<221>  phosphorothioate modification
<222>  (1)..(16)

<400>  30
tcgtcgttct cgtcgt                                                       16

<210>  31
<211>  22
<212>  DNA
<213>  Artificial Sequence

<220>
<223>  IDX-9153


<220>
<221>  phosphorothioate modification
<222>  (1)..(22)

<400>  31
aacgacgatg gcagaacgac ga                                                22


<210>  32
<211>  22
<212>  DNA
<213>  Artificial Sequence

<220>
<223>  IDX-9146


<220>
<221>  phosphorothioate modification
<222>  (1)..(22)

<400>  32
aagcagcatg gcagaagcag ca                                                22


<210>  33
<211>  21
<212>  DNA
<213>  Artificial Sequence

<220>
<223>  IDX-9147


<220>
<221>  phosphorothioate modification
<222>  (1)..(21)

<400>  33
tcgtcgttcg tcgttcgtcg t                                                 21


<210>  34
<211>  21
<212>  DNA
<213>  Artificial Sequence

<220>
<223>  IDX-9148


<220>
<221>  phosphorothioate modification
<222>  (1)..(21)

<400>  34
ttctcgttct gccatcgtga t                                                 21


<210>  35

<211>  16
<212>  DNA
<213>  Artificial Sequence

<220>
<223>  IDX-9149


<220>
<221>  phosphorothioate modification
<222>  (1)..(16)

<400>  35
tcgttccgcc gatcgt                                                    16


<210>  36
<211>  23
<212>  DNA
<213>  Artificial Sequence

<220>
<223>  IDX-9150


<220>
<221>  phosphorothioate modification
<222>  (1)..(23)

<400>  36
tcgtcgttct gccattcgtc cat                                            23


<210>  37
<211>  23
<212>  DNA
<213>  Artificial Sequence

<220>
<223>  IDX-9151


<220>
<221>  phosphorothioate modification
<222>  (1)..(23)

<400>  37
tcgtccattc tgccatcgtc gtt                                            23


<210>  38
<211>  22
<212>  DNA
<213>  Artificial Sequence

<220>
<223>  IDX-9152


<220>
<221>  phosphorothioate modification
<222>  (1)..(22)

<400>  38
tcgtcgttct gaagtcgtcg tt                                             22


<210>  39
<211>  6

<212>  DNA
<213>  Artificial Sequence

<220>
<223>  IDX-0491


<220>
<221>  phosphorothioate modification
<222>  (1)..(6)

<400>  39
tggggt                                                                    6


<210>  40
<211>  11
<212>  DNA
<213>  Artificial Sequence

<220>
<223>  IDX-0492


<220>
<221>  phosphorothioate modification
<222>  (1)..(11)

<400>  40
gcggtttgcg g                                                             11


<210>  41
<211>  15
<212>  DNA
<213>  Artificial Sequence

<220>
<223>  IDX-0493


<220>
<221>  phosphorothioate modification
<222>  (1)..(15)

<400>  41
ggttggtgtg gttgg                                                         15


<210>  42
<211>  7
<212>  DNA
<213>  Artificial Sequence

<220>
<223>  IDX-0494


<220>
<221>  phosphorothioate modification
<222>  (1)..(7)

<400>  42
gcatgct                                                                   7


<210>  43
<211>  24
<212>  DNA

<213>  Artificial Sequence

<220>
<223>  IDX-0495


<220>
<221>  phosphorothioate modification
<222>  (1)..(24)

<400>  43
gcctactaag taatgactgt catg                                              24


<210>  44
<211>  22
<212>  DNA
<213>  Artificial Sequence

<220>
<223>  IDX-9022


<220>
<221>  phosphorothioate modification
<222>  (1)..(22)

<400>  44
tcgtcgttct gccatcgtcg tt                                                22


<210>  45
<211>  27
<212>  DNA
<213>  Artificial Sequence

<220>
<223>  IDX-9012


<220>
<221>  phosphorothioate modification
<222>  (1)..(27)

<400>  45
tcgttcgtct tgttcgtctt gttcgtc                                           27


<210>  46
<211>  18
<212>  DNA
<213>  Artificial Sequence

<220>
<223>  IDX-9010


<220>
<221>  phosphorothioate modification
<222>  (1)..(18)

<400>  46
tcccaagagt cgtccagg                                                     18


<210>  47
<211>  21
<212>  DNA
<213>  Artificial Sequence

<220>
<223>  IDX-9071


<220>
<221>  phosphorothioate modification
<222>  (1)..(21)

<400>  47
tcgttcgtct gcttgttcgt c                                                21


<210>  48
<211>  24
<212>  DNA
<213>  Artificial Sequence

<220>
<223>  IDX-0001


<220>
<221>  phosphorothioate modification
<222>  (1)..(24)

<400>  48
tccgcgttcg gcctcctggc gcgg                                             24


<210>  49
<211>  23
<212>  DNA
<213>  Artificial Sequence

<220>
<223>  IDX-9024


<220>
<221>  phosphorothioate modification
<222>  (1)..(23)

<400>  49
tgccattcgt cgttctcgtc gtt                                              23


<210>  50
<211>  21
<212>  DNA
<213>  Artificial Sequence

<220>
<223>  IDX-9038


<220>
<221>  phosphorothioate modification
<222>  (1)..(21)

<400>  50
tcgtcgttcg gccgatcgtc c                                                21


<210>  51
<211>  19
<212>  DNA
<213>  Artificial Sequence

<220>
<223>  IDX-9074


<220>
<221>  phosphorothioate modification
<222>  (1)..(19)

<400>  51
tcgttcgtct ttcgtctgc                                                    19


<210>  52
<211>  22
<212>  DNA
<213>  Artificial Sequence

<220>
<223>  IDX-9087


<220>
<221>  phosphorothioate modification
<222>  (1)..(22)

<400>  52
tcgtctgctt agttcgttag tt                                                22


<210>  53
<211>  23
<212>  DNA
<213>  Artificial Sequence

<220>
<223>  IDX-9092


<220>
<221>  phosphorothioate modification
<222>  (1)..(23)

<400>  53
tttcgtctgc tttcgtttcg ttt                                               23


<210>  54
<211>  18
<212>  DNA
<213>  Artificial Sequence

<220>
<223>  IDX-9095


<220>
<221>  phosphorothioatem odification
<222>  (1)..(18)

<220>
<221>  phosphorothioate modification
<222>  (1)..(18)

<400>  54
tcgtctgctt tcgtctgc                                                     18


<210>  55
<211>  16

<212>  DNA
<213>  Artificial Sequence

<220>
<223>  IDX-9096


<220>
<221>  phosphothiorate modification
<222>  (1)..(16)

<400>  55
gatcgtccga tcgtcc                                                    16


<210>  56
<211>  24
<212>  DNA
<213>  Artificial Sequence

<220>
<223>  IDX-0910


<220>
<221>  phosphorothioate modification
<222>  (1)..(24)

<400>  56
tcgtcgtttt gtcgttttgt cgtt                                           24


<210>  57
<211>  24
<212>  DNA
<213>  Artificial Sequence

<220>
<223>  IDX-0912


<220>
<221>  phosphorothioate modification
<222>  (1)..(24)

<400>  57
tcgtcgtttt gtcgttttgt cgtt                                           24


<210>  58
<211>  21
<212>  DNA
<213>  Artificial Sequence

<220>
<223>  IDX-9009


<220>
<221>  phosphorothioate modification
<222>  (1)..(21)

<400>  58
tgcgcgcggc ctctcctcgc c                                              21


<210>  59
<211>  15
<212>  DNA

<213>  Artificial Sequence

<220>
<223>  IDX-0465


<220>
<221>  phosphorothioate modification
<222>  (1)..(15)

<400>  59
ggggtgctct gcggg                                                        15


<210>  60
<211>  21
<212>  DNA
<213>  Artificial Sequence

<220>
<223>  IDX-9008


<220>
<221>  phosphorothioate modification
<222>  (1)..(21)

<400>  60
tctgtcgtgt ccttctttgg c                                                 21


<210>  61
<211>  24
<212>  DNA
<213>  Artificial Sequence

<220>
<223>  IDX-9018


<220>
<221>  phosphorothioate modification
<222>  (1)..(24)

<400>  61
tcgtcgtctg aagccgcgcg cggc                                              24


<210>  62
<211>  20
<212>  DNA
<213>  Artificial Sequence

<220>
<223>  IDX-9019


<220>
<221>  phosphorothioate modification
<222>  (1)..(20)

<400>  62
tcgtcgtctg aagccgcggc                                                   20


<210>  63
<211>  23
<212>  DNA
<213>  Artificial Sequence

<220>
<223>  IDX-9020


<220>
<221>  phosphorothioate modification
<222>  (1)..(23)

<400>  63
ctgaagccgc ggctttcgtc gtt                                               23


<210>  64
<211>  20
<212>  DNA
<213>  Artificial Sequence

<220>
<223>  IDX-9021


<220>
<221>  phosphorothioate modification
<222>  (1)..(20)

<400>  64
ctgaagccgg cttcgtcgtt                                                   20


<210>  65
<211>  23
<212>  DNA
<213>  Artificial Sequence

<220>
<223>  IDX-9023


<220>
<221>  phosphorothioate modification
<222>  (1)..(23)

<400>  65
tcgtcgattc tgaagtcgtc gtt                                               23


<210>  66
<211>  22
<212>  DNA
<213>  Artificial Sequence

<220>
<223>  IDX-9026


<220>
<221>  phosphorothioate modification
<222>  (1)..(22)

<400>  66
gttcgtcgat ggccggccgg cc                                                22


<210>  67
<211>  17
<212>  DNA
<213>  Artificial Sequence

<220>
<223>  IDX-9027


<220>
<221>  phosphorothioate modification
<222>  (1)..(17)

<400>  67
ttcgtcgatg gccggcc                                                      17


<210>  68
<211>  23
<212>  DNA
<213>  Artificial Sequence

<220>
<223>  IDX-9039


<220>
<221>  phosphorothioate modification
<222>  (1)..(23)

<400>  68
ggggtcgtct gctatcgatg ggg                                               23


<210>  69
<211>  23
<212>  DNA
<213>  Artificial Sequence

<220>
<223>  IDX-9040


<220>
<221>  phosphorothioatemodification
<222>  (1)..(23)

<400>  69
ggggtcgtct gcgatcgatg ggg                                               23


<210>  70
<211>  24
<212>  DNA
<213>  Artificial Sequence

<220>
<223>  IDX-9041


<220>
<221>  phosphorothioate modification
<222>  (1)..(24)

<400>  70
ggtcgtctgc gacgatcgtc gggg                                              24


<210>  71
<211>  24
<212>  DNA
<213>  Artificial Sequence

<220>

<223>  IDX-9042


<220>
<221>  phosphorothioate modification
<222>  (1)..(24)

<400>  71
cctcgtctgc gacgatcgtc gggg                                              24


<210>  72
<211>  16
<212>  DNA
<213>  Artificial Sequence

<220>
<223>  IDX-9047


<220>
<221>  phosphorothioate modification
<222>  (1)..(16)

<400>  72
ggggtcgtct gctggg                                                       16


<210>  73
<211>  16
<212>  DNA
<213>  Artificial Sequence

<220>
<223>  IDX-9048


<220>
<221>  phosphorothioate modification
<222>  (1)..(16)

<400>  73
ggggtcgtct gcgggg                                                       16


<210>  74
<211>  17
<212>  DNA
<213>  Artificial Sequence

<220>
<223>  IDX-9049


<220>
<221>  phosphorothioate modification
<222>  (1)..(17)

<400>  74
ggggtcgtct gctcggg                                                      17


<210>  75
<211>  17
<212>  DNA
<213>  Artificial Sequence

<220>
<223>  IDX-9050

<220>
<221>  phosphorothioate modification
<222>  (1)..(17)

<400>  75
ggggtcgtct gccaggg                                                      17


<210>  76
<211>  20
<212>  DNA
<213>  Artificial Sequence

<220>
<223>  IDX-9055


<220>
<221>  phosphorothioate modification
<222>  (1)..(20)

<400>  76
gatcgtccgg gtcccggggg                                                   20


<210>  77
<211>  15
<212>  DNA
<213>  Artificial Sequence

<220>
<223>  IDX-9057


<220>
<221>  phosphorothioate modification
<222>  (1)..(15)

<400>  77
gatcgtccgc ggggg                                                        15


<210>  78
<211>  21
<212>  DNA
<213>  Artificial Sequence

<220>
<223>  IDX-9067


<220>
<221>  phosphorothioate modification
<222>  (1)..(21)

<400>  78
tcgtctgcca tggcggccgc c                                                 21


<210>  79
<211>  21
<212>  DNA
<213>  Artificial Sequence

<220>
<223>  IDX-9068

<220>
<221>  phosphorothioate modification
<222>  (1)..(21)

<400>  79
tcgtctgcca tggcgcgccg g                                                21


<210>  80
<211>  12
<212>  DNA
<213>  Artificial Sequence

<220>
<223>  IDX-9133


<220>
<221>  phosphorothioate modification
<222>  (1)..(12)

<400>  80
gatcgtccgt gt                                                          12


<210>  81
<211>  15
<212>  DNA
<213>  Artificial Sequence

<220>
<223>  IDX-9054


<220>
<221>  phosphorothioate modification
<222>  (1)..(15)

<400>  81
ggggtcgtct gcggg                                                       15


<210>  82
<211>  15
<212>  DNA
<213>  Artificial Sequence

<220>
<223>  IDX-0440

<400>  82
ggggtcgtct gcggg                                                       15


<210>  83
<211>  12
<212>  DNA
<213>  Artificial Sequence

<220>
<223>  IDX-9059


<220>
<221>  phosphorothioate modification
<222>  (1)..(12)

<400>  83

gatcgtccgg gg                                                          12

<210>  84
<211>  15
<212>  DNA
<213>  Artificial Sequence

<220>
<223>  IDX-9004

<220>
<221>  phosphorothioate modification
<222>  (1)..(15)

<400>  84
ggggtcgcag ctggg                                                       15

<210>  85
<211>  25
<212>  DNA
<213>  Artificial Sequence

<220>
<223>  IDX-9005

<220>
<221>  phosphorothioate modification
<222>  (1)..(25)

<400>  85
tcgtccatgg tcagggtccc ggggg                                            25

<210>  86
<211>  21
<212>  DNA
<213>  Artificial Sequence

<220>
<223>  IDX-9030

<220>
<221>  phosphorothioate modification
<222>  (1)..(21)

<400>  86
tcgtctgcca tggcggccgc c                                                21

<210>  87
<211>  15
<212>  DNA
<213>  Artificial Sequence

<220>
<223>  IDX-9060

<220>
<221>  phosphorothioate modification
<222>  (1)..(15)

<400>  87
ggggatcgtc cgggg                                                       15

<210>  88
<211>  13
<212>  DNA
<213>  Artificial Sequence

<220>
<223>  IDX-9045


<220>
<221>  phosphorothioate modification
<222>  (1)..(13)

<400>  88
gggtcgcagc tgg                                                          13


<210>  89
<211>  13
<212>  DNA
<213>  Artificial Sequence

<220>
<223>  IDX-9053


<220>
<221>  phosphorothioate modification
<222>  (1)..(13)

<400>  89
gggtcgtctg cgg                                                          13


<210>  90
<211>  16
<212>  DNA
<213>  Artificial Sequence

<220>
<223>  IDX-9058


<220>
<221>  phosphorothioate modification
<222>  (1)..(16)

<400>  90
gatcgtccgt cggggg                                                       16


<210>  91
<211>  15
<212>  DNA
<213>  Artificial Sequence

<220>
<223>  IDX-0445


<220>
<221>  phosphorothioatemodification
<222>  (1)..(15)

<220>
<221>  phosphorothioate modification
<222>  (1)..(15)

<400>  91
ggggtcgtct gcggg                                                        15


<210>  92
<211>  15
<212>  DNA
<213>  Artificial Sequence

<220>
<223>  IDX-0455


<220>
<221>  phosphorothioate modification
<222>  (1)..(15)

<400>  92
ggggccgtct gcggg                                                        15


<210>  93
<211>  15
<212>  DNA
<213>  Artificial Sequence

<220>
<223>  IDX-0460


<220>
<221>  phosphorothioate modification
<222>  (1)..(15)

<400>  93
gggcccgtct gcggg                                                        15


<210>  94
<211>  12
<212>  DNA
<213>  Artificial Sequence

<220>
<223>  IDX-9134


<220>
<221>  phosphorothioate modification
<222>  (1)..(12)

<400>  94
gatgctctgg gg                                                           12

## Description

### FIELD OF THE INVENTION

The present invention relates to methods for identifying oligonucleotides capable of modulating the immune system in a mammalian subject, comprising analysis of which tertiary structural form said oligonucleotide adopts, in aqueous solution. Further, the invention provides oligonucleotides identifiable by the methods of the invention and to their use in methods of treating diseases, such as infectious diseases, inflammatory diseases, neurodegenerative diseases and cancer.

### BACKGROUND

DNA oligonucleotides (oligodeoxyribonucleotides, oligodeoxyribonucleic acids, ODNs, oligonucleotides) are short DNA-based synthetic polymers that can be synthesised and highly purified in significant quantities. The sequence of monomers (deoxyribonucleotides) in oligonucleotides is termed as the primary structure of DNA. The secondary structure of a nucleic acid molecule refers to the base-pairing interactions within a single molecule or set of interacting molecules. The tertiary structure of DNA is determined as its spatial organization (IUPAC). ODNs in physiologically relevant aqueous solutions are considered to be in random-coiled single-stranded form or in the tertiary structure of double-stranded DNA helix.

The double helix is the dominant tertiary structure for biological DNA that can be in one of three DNA conformations and are believed to be found in nature, A-DNA, B-DNA, and Z-DNA. The B-form described by Watson and Crick is believed to predominate in cells (Richmond T.J., et al. (2003) Nature 423 (6936): 145-150). However several types of nucleic acid structures can be observed that are different from random or classical double-stranded helix forms. Among them are triplexes, quadruplexes and other nucleic acid structures (Soyfer, V.N and Potaman V.N. (1995) Triple-Helical Nucleic Acids. Springer Ver., New York, 360 pp; Burge S., et al. (2006) Nucleic Acids Research, 34,19, 5402-5415).

Recently, it has been found that particular G-rich DNA sequences are capable of forming stable four-stranded structures known as G-quadruplexes (G-quartets) (Burge S., et al. (2006) Nucleic Acids Research, 34,19, 5402-5415; Huppert, J. L. (2008) 37(7):1375-84; Neidle and Balasubramanian (2006) Quadruplex Nucleic Acids, RSC Publishing, Cambridge, UK, 302 pp). G-quartets arise from the association of four adjacent G-bases assembled into a cyclic conformation. These structures are stabilized by von Hoogsteen hydrogen bonding and by base stacking interactions (Skogen M., et al., (2006) BMC Neuroscience 7:65). G-quadruplexes have been shown to be relevant in a number of biological processes (Patel et al., (2007) Nucleic Acids Res. 35(22):7429-55). They are important components of human telomeres (Oganesian L, and Bryan TM (2007) Bioessays 29(2):155-65), and play a role in the regulation of transcription (Qin and Hurley (2008) Biochimie., 90 (8):1149-71; Siddiqui-Jain et al., (2002) Proc Natl Acad Sci USA. (2002) 3;99(18):11593-8) as well as translation (Kumari et al., (2007) Nat Chem Biol. 2007, 3(4):218-21). Parekh-Olmedo et al., ((2004) J Mol Neurosci. 24(2):257-67) showed that certain groups of ODNs can inhibit pathological protein aggregation in Huntington's disease. One of these groups was the G-rich oligonucleotides (GROs). G-quartet formation has also been implicated in the non-antisense antiproliferative effects of GROs. In several cases, the biological effects of oligonucleotides designed as antisense agents were found to be unrelated to inhibition of target protein expression, but instead were associated with the formation of G-quartet structures (Burgess et al., (1995) Proc. Natl. Acad. Sci.USA, 92, 4051-4055; Anselmet, A., et al., (2002) FEBS Lett., 510, 175-180; Benimetskaya, L., et al., (1997) Nucleic Acids Res., 25, 2648-2656; Saijo,Y et al., (1997) Jpn J. Cancer Res., 88, 26-33).

The molecular mechanisms of GRO action are not fully known. One of them appears to be related to the ability of oligonucleotides to bind to nucleolin (Bates, P.J., et al. (1999) J. Biol. Chem., 274, 26369-26377). Binding of nucleolin to other G-quartet-forming sequences such as telomeric DNA, immunoglobulin switch regions and ribosomal genes has also been reported (Dempsey, L.A., et al., (1999) J. Biol. Chem., 274, 1066-1071; Hanakahi, L.A. et al., (1999) J. Biol. Chem., 274, 15908-15912; lshikawa, F. et al., (1999) Mol. Cell. Biol. 13, 4301-4310; Dickinson, L.A. and Kohwi-Shigematsu, T. (1995) Mol. Cell. Biol., 15, 456-465).

Treatment of tumour cells with G-rich oligonucleotides was found to inhibit cell cycle progression by specifically interfering with DNA replication, whereas GRO-treated normal skin cells exhibited minimal perturbation of the cell cycle (Xu X., et al., (2001) J. Biol. Chem. 276, 43221-43230). Further, Antisoma plc, developed G-quadruplex based AS-1411 that is the first oligodeoxyribonucleotide aptamer that reached clinical trials for the potential treatment of cancers, including acute myelogenous leukemia (AML) (Ireson CR and Kelland LR, (2006) Mol Cancer Ther. 5 (12):2957-62; Mongelard F. and Bouvet P., (2006) Curr Opin Mol Ther.12(1): 107-14).

G-rich oligonucleotides can form a variety of possible quadruplex structures, depending on its thermodynamic and kinetic characteristics. Quadruplexes can be formed by one, two or four molecules of oligonucleotides, which are referred to as monomer, dimer and tetramer structures, respectively. (Dapic V., et al., (2003) Nucleic Acids Research 31 (8): 2097-2107).

Circular dichroism (CD) spectroscopy is commonly used to investigate the structure and conformation of nucleic acids (Baase and Johnson Jr. (1979) Nucleic Acids Res., 6(2): 797-814; Giraldo R. et al., (1994) Proc.Natl.Acad.Sci.USA 91: 7658-7662; Hardin C.C. et al., (1991) Biochemistry 30:4460-44721992, Hardin C.C. et al., (1992) Biochemistry 31: 833-841; Paramasivan S, et al. (2007) Methods 43: 324-331) where circular dichroism refers to the differential absorption of left and right circularly polarized light (P. Atkins and J. de Paula (2005) Elements of Physical Chemistry, 4th ed. Oxford University Press). Various DNA quadruplex structures have distinctive circular dichroism spectra (Dapic V, et al., (2003) Nucleic Acids Research 31 (8): 2097-2107) providing the possibility to use selected structures as set of standards or references to compare with CD spectra of oligonucleotides.

The immune system consists of a diverse array of immunocompetent cells and inflammatory mediators that exist in complex networks. These components interact through cascades and feedback circuits, maintaining physiologic inflammation and immunosurveillance. In various autoimmune and allergic dieases, a foreign antigen or autoantigen might upset this fine balance, leading to dysregulated immunity, persistent inflammation, and ultimately pathologic sequel. In recent years, there has been tremendous progress delineating the specific components of the immune system that contribute to various aspects of normal immunity and specific disease states. This has introduced the possibility to treat diseases with immunomodulating substances as protein therapeutics, including monoclonal antibodies and cytokines, which became mainstream treatments in a number of clinical settings.

Imbalances in the cytokine cascade can help the initiation and propagation of the immune driven inflammation. In several inflammatory diseases, including rheumatoid arthritis and inflammatory bowel disease, the proinflammatory cytokine TNF-α has been shown to play a central role in inflammatory reactions and has proven to be an especially attractive target for biological agents. Immunomodulatory cytokines considered of significance in the treatment of infectious diseases, malignancies and autoimmune diseases including interferon type I (IFN-α and IFN-β), IFN-γ and IL-10.

Interferons (IFNs) are cytokines that may be released in response to viruses, bacteria, parasites and tumor cells. Interferons possess immunoregulatory, antiviral and anti-cancer properties. They have been used to successfully treat a number of chronic inflammatory disorders including multiple sclerosis (Paolicelli, D et al., (2009) Targets & Therapy; 3, 369-76), chronic viral hepatitis (Hoofnagel JH and Seeff LB, (2006) N. Eng. J. Med., 355: 2444-51; Chevaliez S and Pawlotsky JM, (2009) Handbook of Experimental Pharmacology, Antiviral Strategies, 189: 203-41) and also in neoplastic diseases (Gill PS et al., (1995) N. Eng. J. Med., 332:1744-8). There are two main classes of IFNs: Type I IFNs (α,β,ε,o,κ) are central in the host defense against pathogens such as viruses whereas type II IFN (γ) mainly contributes to the T-cell-mediated regulation of the immune responses.

IFN-α is produced by the cells of the immune system in response to the presence of a foreign antigen, inducing cell activation of macrophages and natural killer cells and enhancing antigen presentation. There are existing 13 subtypes of IFN-α whereby the two subtypes IFN-α2a and IFN-α2b have been used therapeutically with similar results in hepatitis C (Welzel TM et al., (2009) Hepatology, 49: 1847-58) and renal carcinoma (Coppin C et al., (2008) The Cochrane Collaboration, Targeted therapy for advanced renal cell carcinoma, 1-38). The side effects can, however, be significant with up to 68% of patients presenting with psychiatric symptoms, such as depression, irritability, and insomnia.

IFN-β is produced mainly in fibroblasts and plasmacytoid dendritic cells and is 45% identical to IFN-α sharing similar antiviral activity. Clinically, it has been used in the treatment of MS because of its additional anti-inflammatory effect (Durelli L et al., (2009) Ann Neurol, 65: 499-509). Currently, recombinant IFN-β is used as a first-line treatment for relapsing-remitting form of the MS disease. Common adverse events are depression and suicidal ideation, flu-like symptoms, and increase of liver enzyme levels. However, treatment results in the induction of anti-IFN-β neutralizing antibodies (NAbs) in some patients resulting in a lost effect of treatment (Soelberg Sorensen P et al., (2003) Lancet, Vol. 362: 1184-91; Soelberg Sorensen P et al., (2006) Neurology, 67: 1681-3). IFN-β was also used successfully as therapy in chronic inflammatory diseases as ulcerative colitis (Musch E et al., (2002) Aliment Pharmacol Ther, 3: 581-5).

IFN-γ is produced by leukocytes to induce macrophage activation and increase oxidative burst. Defects in IFN-γ and IFN-γ receptor genes have been associated with autoimmune diseases such as rheumatoid arthritis, type1 diabetes and multiple sclerosis (Chen J and Liu X, (2009) Cellular Immunology, Vol. 254: 85-90). However, treatment of autoimmune diseases supplementing with IFN-γ was ambivalent due to its broad biological effects causing unwanted activities. Further, it is clinically used to enhance immunity in patients with chronic granulomatous disease with good efficacy. Potential side effects include fever, hypotension, and flu-like symptoms (Holland SM, (2009) Clinic Rev Allerg Immunol, 38: 3-10). It is also thought to be beneficial as treatment for brain tumor immunotherapy (Haque A et al., (2007) Neurochem Res, 32: 2203-2209).

Interleukins are a group of multifunctional cytokines that are produced by a variety of lymphoid and non-lymphoid cells of the immune system to mediate communication between the immune cells and are particularly important to promote immune responses as inflammation and in the hematopoeisis. An example of a proinflammatory classified interleukin is IL-6. Its dysregulation can contribute to the induction and maintenance of several diseases such as rheumatoid arthritis and inflammatory bowel disease (Heinrich PC et al., (2003) Biochem. J., 1374: 1-20). IL-6 has also anti-inflammatory properties by for example inhibiting TNFs (Opal SM and DePalo VA, (2000) Chest Anti-inflammatory cytokines, 117: 932-4) reflecting the challenge of using a cytokine as therapy or as target for immunotherapy. In contrast, IL-10 is classified as an anti-inflammatory cytokine and is produced by monocytes, macrophages, mast cells, T and B lymphocytes, and dendritic cells. It is believed that it can suppress the production of pro-inflammatory cytokines and plays a central role in the regulation of immune responses. It also has broad implications in the development of certain inflammatory diseases, most noticeably allergy and asthma (Hawrylowicz CM and O'Garra A, (2005) Nat Rev lmmunol, 202: 1459-63; Ogawa Y et al., (2008) Curr Mol Med, 8: 437-45). Numerous clinical studies have indicated that there is a general lack of sufficient levels of IL-10 in asthmatic patients which may contribute to a more intensive inflammation as shown by K. Tomita and colleagues who described that levels of IL-10 and IL-10 producing cells were significantly reduced in patients with severe persistent asthma when compared to mild asthma (Tomita K et al., (2002) Clin Immunol, 102: 258-66). It is also believed that corticosteroids, widely used anti-inflammatory compounds, exert their anti-inflammatory effects in part by enhancing IL-10 production (Richards DF et al., (2000) Eur J lmmunol, 30: 2344-54). In corticosteroid resistant asthmatic patients, corticoids failed to induce IL-10 synthesis suggesting a strong link between induction of IL-10 synthesis and efficacy of corticosteroids (Hawrylowicz CM et al., (2002) J Allergy Clin Immunol, 109: 369-70). Experiments from D. Hesse and colleagues (Hesse D et al., (2010) Europ. J. Neurol., 15: 1-7) indicated that the expression of endogenous IFN-β induces the expression of IL-10 in MS and that the expression of IL-10 negatively correlates with the disease activity suggesting that IL-10 expression is associated with the dampening of the inflammatory response. Further, in neutralizing antibodies (NAbs), positive patients IL-10 expression is reduced.

The use of cell surface antigens as therapeutic targets is another growing area of modulating the immune system. Using antibody-related therapies can have several options such as binding to a specific target molecule on the cell surface to trigger cellular mechanisms such as apoptosis or activation pathways (immunotherapy), or simply bind to a target on the cell surface for delivery of an agent to the specific cell type, e.g. cytostatic agent (immune-chemotherapy). Immunotherapy is used in the treatment or alleviation of many immunological diseases or conditions, such as cancer, inflammatory diseases such as asthma and allergy and also autoimmune disorders such as multiple sclerosis.

WO2010/053433 A1 describes the potential of specific oligonucleotides in up-regulating the expression of certain cell surface markers or cell surface antigens as CD20, CD23, CD69 and CD80. The pre-incubation of PBMC isolated from CLL patients significantly increased the rate of apoptosis in human B-cells mediated by a monoclonal antibody directed against CD20 (rituximab).

WO2010/053430 A1 describes the capability of specific oligonucleotides to influence the properties and behaviour of polymorphonuclear cells, in particular the recruitment and/or migration of polymorphonuclear cells to a site of inflammation, and that they through this mechanism have utility in the prevention, treatment and/or alleviation of various diseases such as ischemia.

The challenges of immunotherapy and treatment with cytokines are the occurring side effects and the observed immunogenicity of these protein therapeutics even of fully human protein drugs (Vial T and Descotes J, (1994) Drug Saf, 10: 115-20; Scott DW and De Groot AS, (2010) ANN Rheum Dis, 69: 72-76). Especially, for the treatment with IFN-α it was suggested that the efficacy of the treatment has to be increased while the toxicity should be decreased (Sarasin-Filipowicz M, (2010) Swiss Med Wkly, 140: 3-11). The usage of endogenous induced IFN-α could be more effective and tolerable. P. Sfriso and colleagues could for example show that exposure to fungi is positive for the treatment of inflammatory bowel disease (Sfriso P et al., (2010) J Leuk Biol, 87: 385-95). Fungi are a natural source of foreign DNA and proteins, inducing endogenous cytokine production. Endogenous induction of cytokines could give beneficial effects without unwanted induced activities. Another aspect is that IFN-α, for example, exists in numerous subforms, however through endogenously induced expression all subforms will be expressed in their natural way. It could be shown in corticosteroid-resistant asthma patients that IL-10 is up-regulated after IFN-α treatment and the authors suggest that the beneficial effects of IFN-α lies in the production of IL-10 (Simon HU et al., (2003) Allergy, 58: 1250-1255). IL-10 has less side effects than IFN-α and therefore could be a better treatment option. Chen and collegues described the opposite functions of IL-10 and IFN-γ in a subform of CD4+ T-cells while they are working together in the disease management of chronic infections (Chen J and Liu XS, (2009) J Leuk Biol, 86: 1305-10). These examples demonstrate how different cytokines with different biological functions can act together to modulate the pathogenesis of a disease or to maintain the fine balance in an immune response. A more effective treatment option could be a combination of different cytokines or a way to induce different cytokines endogenously.

Although synthesized oligonucleotides are interacting with the same cellular receptor (TLR9), the inventors have found surprisingly that various oligonucleotides can induce different patterns of cytokine expression in human PBMCs and this is dependent on the tertiary structure of the oligonucleotides and not from the content of any specific sequence feature as for example the dinucleotide CpG. Non-CpG oligonucleotides can induce cytokine expression and some CpG containing oligonucleotides cannot. The inventors have developed a method with which they can identify new oligonucleotides that can induce preferably specific cytokines dependent on their teriary structure and could be used for the treatment of diseases known to be deficient in these cytokines.

There is clearly a need to provide methods and oligonucleotides that can induce the endogenous expression of specific cytokines.

### DESCRIPTION OF THE INVENTION

This description is not to be taken in a limiting sense, but is made solely for the purpose of describing the general principles of the invention. The scope of the invention should be determined with reference to the claims.

The inventors have set out to develop a method for identification of novel oligonucleotides capable of endogenously modulating the immune system in a mammalian subject depending on the tertiary structure adopted in solution and, therefore useful in the treatment of inflammatory and autoimmune diseases as well as in infectious and neurodegenerative diseases and cancer. Other objects underlying the invention, as well as advantages associated with the invention, will become evident to the skilled person upon study of the description, examples and claims.

The inventors have surprisingly identified by tertiary structure analysis that dependent on which tertiary structure an oligonucleotide adopts in aqueous solution, it influences the degree and type of immunomodulation in mammalian subject. The cytokine profile release of cultured peripheral blood mononuclear cells in the presence of certain oligonucleotides, has been shown to be dependent on the particular spatial three-dimensional structure of said oligonucleotides. When a nucleotide, in solution, comprises a certain amount in a particular tertiary form, they are surprisingly useful in modulating the immune system, including increasing levels of cytokines, cell surface markers, receptors, prostaglandins and hormones. Such oligonucleotides are potentially useful in the treatment of diseases, wherein a modulation of the immune system is of benefit for the treatment of a disease. Accordingly, the invention provides methods for identifying substances and, oligonucleotides in particular, that are capable of modulating the immune system in a mammalian subject, by analysis of which tertiary structural form said oligonucleotide adopts, in aqueous solution.

### Short description of the drawings

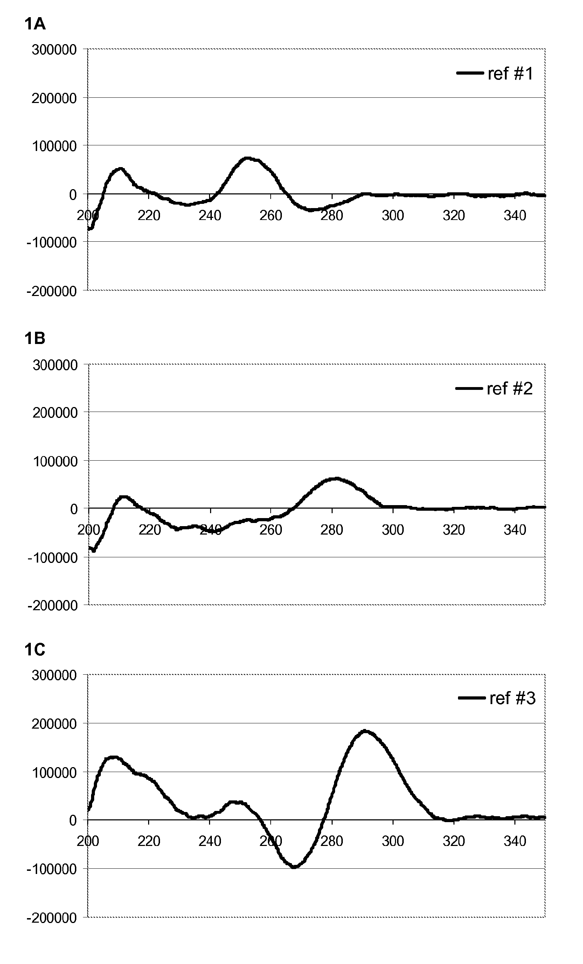

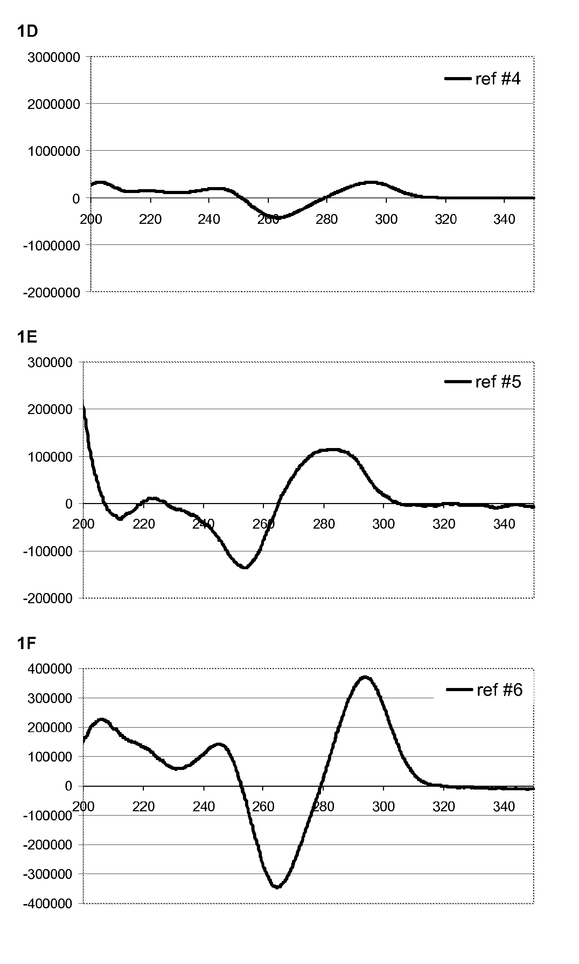

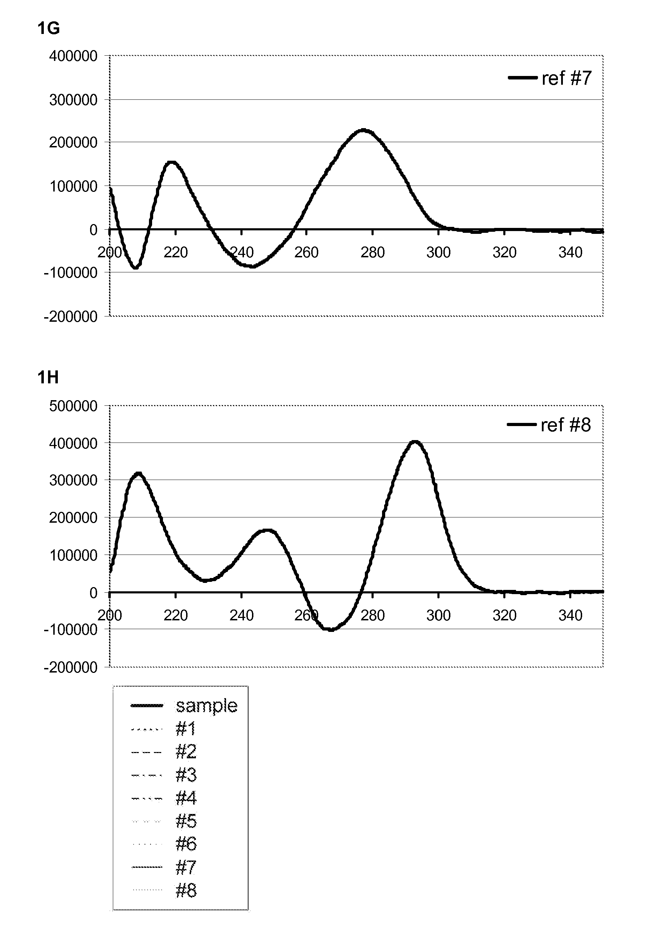

Figure 1 depicts the aligned CD spectra of reference oligonucleotides used in the calculation of the relative structural composition of the CD spectra of the oligonucleotides of the invention by measurement of amount of tertiary structural form as compared to the level of tertiary structural form of a reference oligonucleotide, in aqueous solution. The wavelength of the spectra is represented on the abscissa axis and corresponding molar ellipticity value is represented on the ordinate axis.

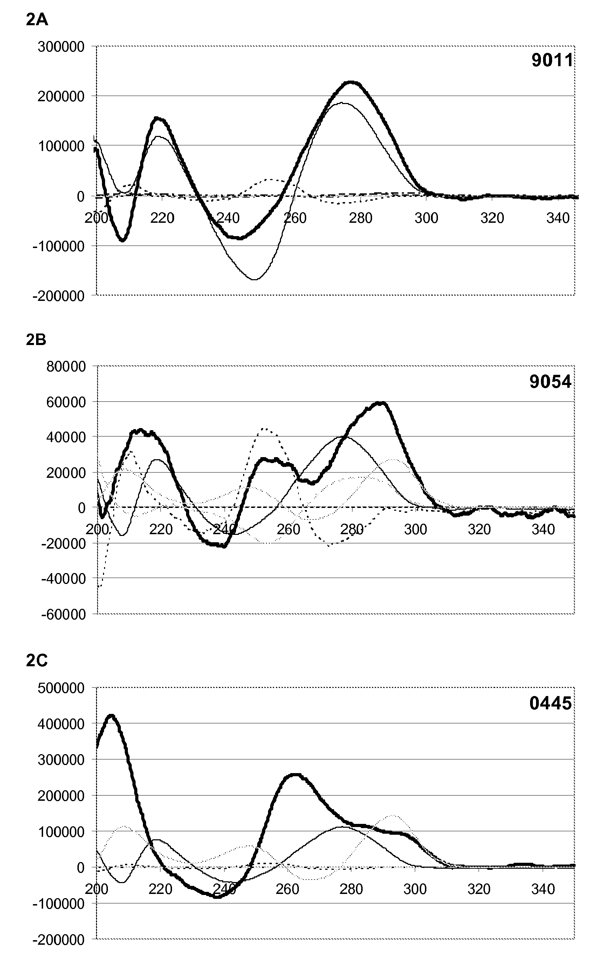

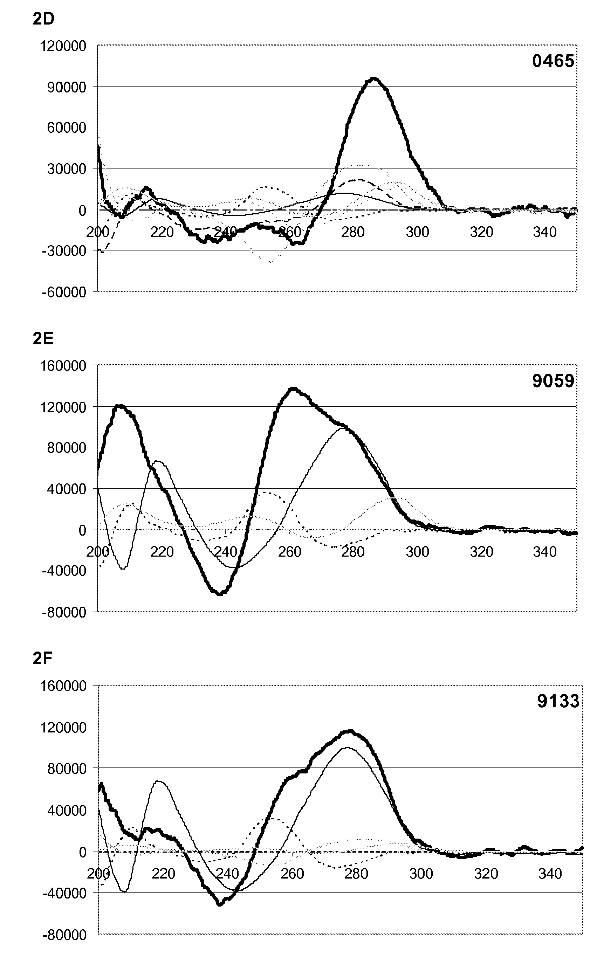

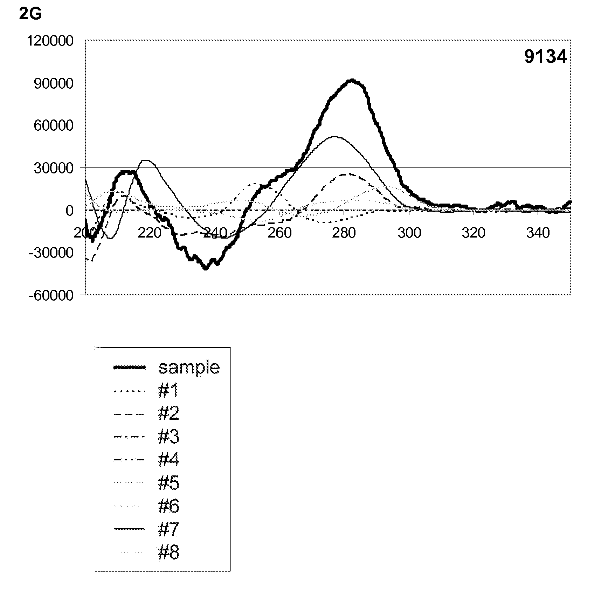

Figure 2 depicts the molar ellipticity of 7 samples of oligonucleotides with experimental data overlaid on the calculated CD spectra input of standard components; CD spectra indicated as follows: sample - experimental measured spectra of oligonucleotide, #1 - standard component 1 (SEQ ID NO 1); #2 - standard component 2 (SEQ ID NO 2); #3 - standard component 3 (SEQ ID NO 3); #4 -standard component 4 (SEQ ID NO 4); #5 - standard component 5 (SEQ ID NO 5); #6 - standard component 6 (SEQ ID NO 6); #7 - standard component 7 (SEQ ID NO 7); #8 - standard component 8 (SEQ ID NO 8). The samples represent oligonucleotides with SEQ ID NO 7 (IDX-9011); SEQ ID NO 81 (IDX-9054); SEQ ID NO 91 (IDX-0445); SEQ ID NO 59 (IDX-0465); SEQ ID NO 83 (IDX-9059); SEQ ID NO 80 (IDX-9133); and SEQ ID NO 94 (IDX-9134). The wavelength of the spectra is represented on the abscissa axis and corresponding molar ellipticity value is represented on the ordinate axis.

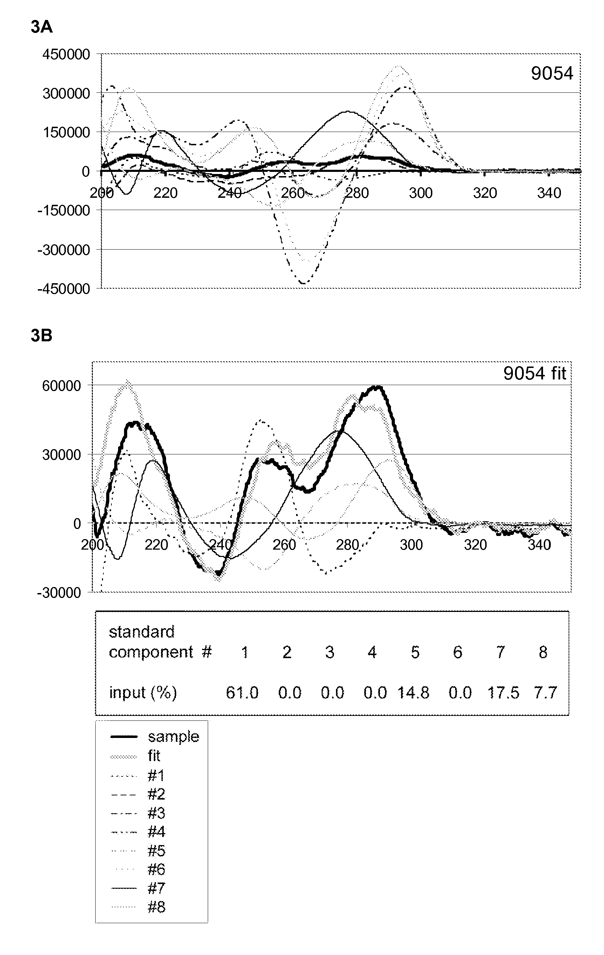

Figure 3 represents an example of calculation of the relative composition of the CD spectra of IDX 9054 (SEQ ID NO 81) applying a fitting analysis using computer program for the mathematical decomposition of experimental data. **3A**: Molar ellipticity of IDX 9054 (SEQ ID NO 81) overlaid on the aligned experimental measured CD spectra of the oligonucleotides used as standard components in the calculation of the relative structural composition of the CD spectra of the samples. **3B**: Molar ellipticity of IDX 9054 (SEQ ID NO 81) overlaid on the aligned theoretically calculated fitting CD spectra of standard components and on the fitting curve of sum of the theoretically calculated CD spectra of standard components. Inserted table indicates calculated fitting CD spectra input (%) of standard components. The wavelength of the spectra is on the abscissa axis and corresponding molar ellipticity value is on the ordinate axis.

### DETAILED DESCRIPTION OF THE INVENTION

Before the invention is described in detail, it is to be understood that this invention is not limited to the particular compounds described or process steps of the methods described since compounds and methods may vary. It is also to be understood that the terminology used herein is for purposes of describing particular embodiments only, and is not intended to be limiting. It must be noted that, as used in the specification and the appended claims, the singular forms "a," "an" and "the" include plural referents unless the context clearly dictates otherwise. Thus, for example, reference to "a sequence" includes more than one such sequence, and the like.

Further, the term "about" is used to indicate a deviation of +/- 2 percent of the given value, preferably +/- 5 percent and most preferably +/- 10 percent of the numeric values, when applicable.

The phrase "capable of modulating the immune system" is used to describe a function, wherein a substance is capable to alter an immune response. Immunomodulation is an alteration, adjustment or regulation of the immune response. An immunomodulator is a substance that has an effect on the immune system in form of immunostimulation or immunosupression or both. This effect can be mediated by, but is not limited to, cytokines (lymphokines, chemokines, interleukins, interferons), cell surface markers, receptors, prostaglandins and hormones. These changes in the immune response can be for example measured through the release of cytokines, expression changes of cell surface markers or other physiological parameters as proliferation. The substance or immunomodulator is preferably an oligonucleotide.

The term "oligonucleotide" refers to a nucleic acid polymer, typically with from 5 to 120 bases, preferably of about 12 to about 30 nucleotides. Preferably, said oligonucleotide represents a DNA oligonucleotide, which should be interpreted as being equal to an oligodeoxyribonucleotide or an oligodeoxyribonucleic acid (ODN).

The phrase "tertiary structural form" refers to different spatial organizations that an oligonucleotide may adopt. In relation to the present invention, the following described types of tertiary structural forms are relevant; tetramer/telomere form; dimer/fragile X form; dimer/telomere form 1; dimer/telomer form 2; dimer/non-G-quadruplex form; monomer/basket form; random form and monomer/thrombin form. These structural forms are further described and defined below.

The phrase "adopts, in aqueous solution" refers to that the tertiary structural form of the oligonucleotides of the invention is measureable preferably in an aqueous solution. An aqueous solution refers to a solution in which the primary solvent is water.

The testing of the capability for oligonucleotides of the invention to modulate the immune system in mammalian subjects, may be carried out as described below in Example 2.

The invention relates to the surprising concept that if a certain oligonucleotide forms a certain tertiary structure, at least to a certain percentage in a composition, it is capable of modulating the immune system, such as increasing levels of cytokines. Therefore, there is provided methods for identification of such oligonucleotides. In order to determine the tertiary structure, samples were prepared and analyzed by circular dichroism (CD) measurement. The results were compared to the results of CD measurements of a number of reference oligonucleotides that are capable of, and established to form particular tertiary structures. The sequences of these reference oligonucleotides are disclosed in Table 1.

**Table 1. Structure references for CD spectra analysis**

| **SEQ ID NO** | **Sequence 5'-3'** | **Structure type/form** | **Ref. number** | **IDX-No** |
|---|---|---|---|---|
| 1 | TGGGGT | tetramer/ telomere | #1 | 0400 |
| 2 | GCGGTTTGCGG | dimer/ fragile X | #2 | 0405 |
| 3 | GGGTTTTGGG | dimer/ telomere form 1 | #3 | 0415 |
| 4 | GGGGTTTTGGGG | dimer/ telomer form 2 | #4 | 0420 |
| 5 | GCATGCT | dimer/non-G-quadruplex | #5 | 0430 |
| 6 | GGTTTTGGTTTTGGTTTTGG | monomer/ basket G-quadruplex | #6 | 0435 |
| 7 | T*C*A*CGACCGTCAAAC*T*C*C | random | #7 | 9011 |
| 8 | GGTTGGTGTGGTTGG | monomer/ thrombin | #8 | 0410 |

| | | | | |
|---|---|---|---|---|
| = phosphorothioate modification | | | | |

The properties set out in Table 1, and adhered to throughout this specification, are to be understood as:

### Tetramer/telomere form

Oligonucleotide TGGGGT is derived from the *O. nova* telomeric sequence. Previous work using both X-ray crystallographic (XRC) and nuclear magnetic resonance (NMR) has shown that it forms a tetrameric structure (see e.g. Phillips,K., et al. (1997) J. Mol. Biol., 273, 171-182; Aboul-ela,F. et al. (1994) J. Mol. Biol., 243, 458-471; Aboul-ela,F. et al.(1992) Nature, 360, 280-282.). Thus, the oligonucleotide TGGGGT (SEQ ID NO 1 - reference #1) serves as reference oligonucleotide for a tetramer/telomere form and its CD spectrum is depicted in Figure 1A.

### Dimer/fraqile X form

Oligonucleotide GCGGTTTGCGG represents the fragile X gene repeat sequence and has been shown to form a specific dimeric structure (see Kettani, A. et al. (1995) J. Mol. Biol., 254, 638-65). Thus, the oligonucleotide GCGGTTTGCGG (SEQ ID NO 2 - reference #2) serves as reference oligonucleotide for a dimer/fragile X form and its CD spectrum is depicted in Figure 1B.

### Dimer/telomere form 1

Oligonucleotide GGGTTTTGGG is derived from the *O. nova* telomeric sequence. NMR studies showed that it forms a specific dimeric G-quadruplex structure (see e.g. Scaria,P.V. et al. (1992) Proc. Natl Acad. Sci. USA, 89, 10336-10340; Keniry, M.A. et al. (1995) Eur. J. Biochem., 233, 631-643; Hud,N.V. et al. (1996) Biochemistry, 35, 15383-15390). Thus, the oligonucleotide GGGTTTTGGG (SEQ ID NO 3 - reference #3) serves as reference oligonucleotide for a dimer/telomere form 1 and its CD spectrum is depicted in Figure 1C.

### Dimer/telomer form 2

Oligonucleotide GGGGTTTTGGGG is derived from the *O. nova* telomeric sequence. Both XRC and NMR studies showed that it forms a specific G-dimeric non-GC structure (see e.g. Schultze,P. et al. (1999) Nucleic Acids Res., 27, 3018-3028; Kang,C. et al. (1992) Nature, 356,126-131; Smith,F.W. and Feigon,J. (1993) Biochemistry, 32, 8682-8692; Haider,S. et al. (2002) Mol. Biol., 320, 189-200). Thus, the oligonucleotide GGGGTTTTGGGG (SEQ ID NO 4 - reference #4) serves as reference oligonucleotide for a dimer/telomere form 2 and its CD spectrum is depicted in Figure 1D.

### Dimer/non-G-quadruplex form

Oligonucleotide GCATGCT forms a specific quadruplex dimeric structure that does not involve G-quadruplex formation (see Leonard,G.A. et al. (1995) Structure, 3, 335-340). Thus, the oligonucleotide GCATGCT (SEQ ID NO 5 - reference #5) serves as reference oligonucleotide for a dimer/non-G-quadruplex form and its CD spectrum is depicted in Figure 1E.

### Monomer/basket form

Oligonucleotide GGTTTTGGTTTTGGTTTTGG forms a specific monomeric G-quadruplex structure that was shown using NMR analysis (see Marathias,V.M. and Bolton,P.H. (1999) Biochemistry, 38, 4355-4364). Thus, teh oligonucleotide GGTTTTGGTTTTGGTTTTGG (SEQ ID NO 6 - reference #6) serves as reference oligonucleotide for a monomer/basket form and its CD spectrum is depicted in Figure 1F.

### Random form

Oligonucleotide T*C*A*CGACCGTCAAAC*T*C*C designed by the inventors and is characterised by CD spectroscopy. It shows CD spectra characteristic for DNA that has random coil structure but doesn't form any particular specific tertiary structure. The oligonucleotide T*C*A*CGACCGTCAAAC*T*C*C (SEQ ID NO 7 - reference #7) thus serves as reference oligonucleotide for a random form and its CD spectrum is depicted in Figure 1G.

### Monomer/thrombin form

Oligonucleotide GGTTGGTGTGGTTGG is known as the thrombin-binding aptamer. It was created by an *in vitro* selection approach. It forms a monomeric G-quadruplex structure that was shown by both NMR and crystal structures (see Padmanabhan, K. and Tulinsky, A. (1996) Acta Crystallogr. D, 52, 272-282; Kelly, J.A. et al. (1996) J. Mol. Biol., 256, 417-422; Macaya,R.F. et al. (1993) Proc. Natl Acad. Sci. USA, 90, 3745-3749; Padmanabhan,K. et al. (1993) J. Biol. Chem., 268, 17651-17654). The oligonucleotide GGTTGGTGTGGTTGG (SEQ ID NO 8 - reference #8) thus serves as reference oligonucleotide for monomer/thrombin form and its CD spectrum is depicted in Figure 1H.

In a first aspect of the invention, there is provided a method for identifying an oligonucleotide capable of modulating the immune system in a mammalian subject by analyzing which tertiary structural form said oligonucleotide adopts, in aqueous solution. In said method, said capability of modulating the immune system can be analyzed by testing whether said oligonucleotide modulates the immune system in a mammalian subject. Said testing may be carried out *in vitro*.

In another aspect of the invention, there is provided a method for identifying an oligonucleotide capable of modulating the immune system in a mammalian subject by analyzing which tertiary structural form said oligonucleotide adopts, in aqueous solution, wherein said modulation of the immune system represents an increase of levels of interferons, such as interferon-α, interferon-β and/or interferon-γ.

In one embodiment of this aspect, said oligonucleotide adopts, in aqueous solution, tertiary structural form of at least 40% of tetramer/telomere form. Preferably, said oligonucleotide adopts, in aqueous solution, at least 60%, preferably 80%, more preferably 90% of tetramer/telomere form.

In another embodiment of this aspect, the amount of tertiary structural tetramer/telomere form is measured by comparing the level of tertiary structural form of a reference oligonucleotide that adopts, in aqueous solution, a tetramer/telomere form. Said reference oligonucleotide may represent an oligonucleotide of SEQ ID NO 1.

In another embodiment of this aspect, there is provided an oligonucleotide, identifiable by the methods of the invention. Said oligonucleotide, preferably has at least one nucleotide that has a phosphate backbone modification. Said phosphate backbone modification preferably is a phosphorothioate or phosphorodithioate modification. Said oligonucleotide typically comprises of about 5 to about 120 nucleotides, preferably of about 12 to about 30 nucleotides.

In another aspect of the invention, there is provided a method for identifying an oligonucleotide capable of modulating the immune system in a mammalian subject comprising analyzing which tertiary structural form said oligonucleotide adopts, in aqueous solution, wherein said modulation of the immune system represents an increase or decrease of levels of cell surface markers.

In another aspect of the invention, there is provided a method for identifying an oligonucleotide capable of modulating the immune system in a mammalian subject comprising analyzing which tertiary structural form said oligonucleotide adopts, in aqueous solution wherein said modulation of the immune system represents a change in the properties or behaviour of polymorphonuclear cells.

The methods of the invention have been tested and a number of novel oligonucleotides have been shown to being capable of modulating the immune system. The invention, therefore, makes available specific novel oligonucleotides. Thus, in another aspect of the invention, there is provided isolated oligonucleotides with sequences according to any one of SEQ ID NOs 24 - 43 and SEQ ID NOs 60 - 90, particularly isolated oligonucleotides with sequences according to any one SEQ ID NOs 24 - 43 and SEQ ID NOs 60 - 77 and SEQ ID NOs 79 - 80, preferably isolated oligonucleotides with sequences according to any one of SEQ ID NOs 60 - 77 and SEQ ID NOs 79 - 80. These oligonucleotides have been identified to adopt, in aqueous solution, tertiary structural form at least 40% of tetramer/telomere form. Further, these oligonucleotides have also been shown to being capable of increasing levels of interferons, such as interferon-α, interferon-β and/or interferon-γ. These particular oligonucleotides are presented in Table 2, which correlates the SEQ ID NOs with the nucleotide sequences and corresponding internal reference codes ("IDX-No").

**Table 2. Active oligonucleotides with at least 40% of tetramer/telomere form**

| **SEQ ID NO** | **Sequence 5'-3'** | **IDX-No** |
|---|---|---|
| 24 | T*C*G*TCGTTCTGCCATCGT*C*G*T | 9138 |
| 25 | T*C*G*TCGTTCTCGTC*G*T*T | 9139 |
| 26 | T*C*G*TTCTGCTGAT*C*G*T | 9140 |
| 27 | T*C*G*TCGTTCTGTCGTC*G*T*T | 9141 |
| 28 | T*C*G*TCGTTCGTCGTC*G*T*T | 9142 |
| 29 | T*C*G*TCGTTGCTCGTC*G*T*T | 9143 |
| 30 | T*C*G*TCGTTCTCGT*C*G*T | 9144 |
| 31 | A*A*C*GACGATGGCAGAACGA*C*G*A | 9153 |
| 32 | A*A*G*CAGCATGGCAGAAGCA*G*C*A | 9146 |
| 33 | T*C*G*TCGTTCGTCGTTCGT*C*G*T | 9147 |
| 34 | T*T*C*TCGTTCTGCCATCGT*G*A*T | 9148 |
| 35 | T*C*G*TTCCGCCGAT*C*G*T | 9149 |
| 36 | T*C*G*TCGTTCTGCCATTCGTC*C*A*T | 9150 |
| 37 | T*C*G*TCCATTCTGCCATCGTC*G*T*T | 9151 |
| 38 | T*C*G*TCGTTCTGAAGTCGTC*G*T*T | 9152 |
| 39 | T*G*G*G*G*T | 0491 |
| 40 | G*C*G*GTTTG*C*G*G | 0492 |
| 41 | G*G*T*TGGTGTGGT*T*G*G | 0493 |
| 42 | G*C*A*T*G*C*T | 0494 |
| 43 | G*C*C*TACTAAGTAATGACTGTC*A*T*G | 0495 |
| 60 | T*C*T*GTCGTGTCCTTCTTT*G*G*C | 9008 |
| 61 | T*C*G*TCGTCTGAAGCCGCGCGC*G*G*C | 9018 |
| 62 | T*C*G*TCGTCTGAAGCCGC*G*G*C | 9019 |
| 63 | C*T*G*AAGCCGCGGCTTTCGTC*G*T*T | 9020 |
| 64 | C*T*G*AAGCCGGCTTCGTC*G*T*T | 9021 |
| 65 | T*C*G*TCGATTCTGAAGTCGTC*G*T*T | 9023 |
| 66 | G*T*T*CGTCGATGGCCGGCCG*G*C*C | 9026 |
| 67 | T*T*C*GTCGATGGCCG*G*C*C | 9027 |
| 68 | G*G*G*GTCGTCTGCTATCGATG*G*G*G | 9039 |
| 69 | G*G*G*GTCGTCTGCGATCGATG*G*G*G | 9040 |
| 70 | G*G*T*CGTCTGCGACGATCGTCG*G*G*G | 9041 |
| 71 | C*C*T*CGTCTGCGACGATCGTCG*G*G*G | 9042 |
| 72 | G*G*G*GTCGTCTGCT*G*G*G | 9047 |
| 73 | G*G*G*GTCGTCTGCG*G*G*G | 9048 |
| 74 | G*G*G*GTCGTCTGCTC*G*G*G | 9049 |
| 75 | G*G*G*GTCGTCTGCCA*G*G*G | 9050 |
| 76 | G*A*T*CGTCCGGGTCCCGG*G*G*G | 9055 |
| 77 | G*A*T*CGTCCGCGG*G*G*G | 9057 |
| 78 | T*C*G*T*C*T*G*C*C*A*T*G*G*C*G*G*C*C*G*C*C | 9067 |
| 79 | T*C*G*TCTGCCATGGCGCGC*C*G*G | 9068 |
| 80 | G*A*T*CGTCCG*T*G*T | 9133 |
| 81 | G*G*G*GTCGTCTGC*G*G*G | 9054 |
| 82 | GGGGTCGTCTGCGGG | 0440 |
| 83 | G*A*T*CGTCCG*G*G*G | 9059 |
| 84 | G*G*G*GTCGCAGCT*G*G*G | 9004 |
| 85 | T*C*G*TCCATGGTCAGGGTCCCGG*G*G*G | 9005 |
| 86 | T*C*G*TCTGCCATGGCGGCC*G*C*C | 9030 |
| 87 | G*G*G*GATCGTCCG*G*G*G | 9060 |
| 88 | G*G*G*TCGCAGC*T*G*G | 9045 |
| 89 | G*G*G*TCGTCTG*C*G*G | 9053 |
| 90 | G*A*T*CGTCCGTCGG*G*G*G | 9058 |

| | | |
|---|---|---|
| * = phosphorothioate modification | | |

A number of oligonucleotides having superficially similar nucleotide sequences to the active oligonucleotides in Table 2, are set out in Table 3. These oligonucleotides do not modulate the immune system, in particular, they do not increase levels of interferons in the presented assays. The tertiary structural form of these oligonucleotides were identified and it was shown that they had a different tertiary structural composition than the active oligonucleotides set out in Table 2. For these reasons, the oligonucleotides in Table 3 are designated as control oligonucleotides with little or no contribution of tetramer/telomer form contribution.

**Table 3. Control oligonucleotides with little or no contribution of tetramer/telomere form contribution**

| **SEQ ID NO** | **Sequence 5'-3'** | **IDX-No** |
|---|---|---|
| 91 | G*G*G*G*T*C*G*T*C*T*G*C*G*G*G | 0445 |
| 92 | G*G*G*GCCGTCTGC*G*G*G | 0455 |
| 93 | G*G*G*CCCGTCTGC*G*G*G | 0460 |
| 59 | G*G*G*GTGCTCTGC*G*G*G | 0465 |
| 94 | G*A*T*GCTCTG*G*G*G | 9134 |
| 7 | T*C*A*CGACCGTCAAAC*T*C*C | 9011 |

| | | |
|---|---|---|
| * = phosphorothioate modification | | |

In a further aspect of the invention there is provided an isolated oligonucleotide which adopts, in aqueous solution, a tertiary structure of at least 40% of tetramer/telomere form, said oligonucleotide being capable of modulating the immune system in a mammalian subject upon administration to said subject.

In one embodiment of this aspect, there is provided an oligonucleotide, which adopts, in aqueous solution, a tertiary structure of at least 60%, preferably 80%, more preferably 90% of tetramer/telomere form. Said modulation of the immune system preferably represents an increase in levels of cytokines, such as interferon-α, interferon-β and/or interferon-γ.

In another embodiment of this aspect, said oligonucleotide comprises at least one nucleotide that has a phosphate backbone modification. Preferably, said phosphate backbone modification is a phosphorothioate or phosphorodithioate modification.

In another embodiment of this aspect, said oligonucleotide comprises of about 5 to about 120 nucleotides, preferably of about 12 to about 30 nucleotides.

In another embodiment of this aspect, said oligonucleotide is selected from the group consisting of SEQ ID NO 24 - 43, SEQ ID NO 60 - 77 and SEQ ID NOs 79 - 80.

In another embodiment of this aspect, said oligonucleotide is selected from the group consisting of SEQ ID NO 60 - 77 and SEQ ID NOs 79 - 80.

In another embodiment of this aspect, said oligonucleotide is selected from the group consisting of SEQ ID NO 76, SEQ ID NO 77 and SEQ ID NO 80.

In another embodiment of this aspect, there is provided said oligonucleotide for use in therapy.

In another embodiment of this aspect, there is provided said oligonucleotide for use in treating a disease where increasing the levels of interferons are beneficial for said treatment.

In another embodiment of this aspect, there is provided said oligonucleotide for use in the treatment of diseases selected from infectious diseases, inflammatory diseases, neurodegenerative diseases, autoimmune diseases and cancer.

In another embodiment of this aspect, there is provided said oligonucleotide for use in the treatment of diseases selected from inflammatory bowel disease, hairy cell leukemia, haematological malignancy, multiple sclerosis, hepatitis B, hepatitis C, chronic hepatitis, cirrhosis, chronic granulomatosis disease and severe malignant osteopetrosis.

In another embodiment of this aspect, there is provided use of said oligonucleotide, in the preparation of a medicament useful in the treatment where increasing the levels of interferons are beneficial for said treatment.

In another embodiment of this aspect, there is provided use of said oligonucleotide, in the preparation of a medicament useful in the treatment infectious diseases, inflammatory diseases, neurodegenerative diseases, autoimmune diseases or cancer.

In another embodiment of this aspect, there is provided use of said oligonucleotide, in the preparation of a medicament useful in the treatment of a disease selected from inflammatory bowel disease, hairy cell leukemia, haematological malignancy, multiple sclerosis, hepatitis B, hepatitis C, chronic hepatitis, cirrhosis, chronic granulomatosis disease and severe malignant osteopetrosis.

In another embodiment of this aspect, there is provided use of said oligonucleotide, which use further comprises one or more additional agents effective in treating infectious diseases, inflammatory diseases, neurodegenerative diseases, autoimmune diseases or cancer.

In another embodiment of this aspect, there is provided a method of treating a disease wherein increasing the levels of interferons are beneficial for said treatment, comprising administering to a subject in need thereof, of said oligonucleotide.

In another embodiment of this aspect, there is provided a method of treating infectious diseases, inflammatory diseases, neurodegenerative diseases, autoimmune diseases or cancer, comprising administering to a subject in need thereof, of said oligonucleotide.

In another embodiment of this aspect, there is provided a method of treating a disease selected from inflammatory bowel disease, hairy cell leukemia, haematological malignancy, multiple sclerosis, hepatitis B, hepatitis C, chronic hepatitis, cirrhosis, chronic granulomatosis disease and severe malignant osteopetrosis, comprising administering to a subject in need thereof, of said oligonucleotide.

In another embodiment of this aspect, there is provided a method of treating said disease or diseases, which further comprises one or more additional agents effective in treating infectious diseases, inflammatory diseases, neurodegenerative diseases or cancer.

In another aspect of the invention, there is provided a pharmaceutical composition comprising an oligonucleotide of the invention as well as further oligonucleotides identifiable by the methods of the invention, together with pharmaceutically acceptable adjuvants, diluents or carriers.

According to an embodiment, said oligonucleotide is administered in an amount of about 1 to about 2000 micro g per kg body weight, preferably about 1 to about 1000 micro g per kg body weight. Most preferably the oligonucleotide is administered in an amount of about 1 to 500 micro g per kg body weight.

In a method according to the invention, the route of administration is chosen from mucosal, subcutaneous, intramuscular, intravenous and intraperitoneal administration. According to an embodiment of the method, the mucosal administration is chosen from nasal, oral, gastric, ocular, rectal, urogenital and vaginal administration.

Nasal administration constitutes one embodiment of the method according to the invention. There are several methods and devices available for nasal administration; single or multi-dosing of both liquid and powder formulations, with either topical or systemic action. Using appropriate devices or administration techniques, it is possible to target the olfactory bulb region for delivery to the CNS. The present invention is not limited to particular methods or devices for administering the oligonucleotides to the nasal mucous membrane. The initial animal studies have shown that simple instillation by pipette works satisfactorily, although for human use, devices for reliable single or multi dose of administration would be preferred.

According to another embodiment of the invention, the oligonucleotides are administered to the mucous membrane of the colon through rectal instillation, e.g. in the form of an aqueous enema comprising the oligonucleotides suspended in a suitable buffer.

According to another embodiment of the invention, the oligonucleotides are administered to the mucous membrane of the lungs or the airways through inhalation of an aerosol, comprising the oligonucleotides suspended in a suitable buffer, or by performing a lavage, also comprising the oligonucleotides suspended in a suitable buffer.

According to yet another embodiment of the invention, the oligonucleotides are administered to the mucous membrane of the urogenital tract, such as the urethra, the vagina etc through application of a solution, a buffer, a gel, salve, paste or the like, comprising the oligonucleotides suspended in a suitable vehicle.

A particular embodiment involves the use of an oligonucleotide according to the invention for use in conjunction with the administration of glucocorticosteroids (GCS). There are indications that the oligonucleotides according to the invention can enhance GCS action. The inventors thus make available a combination therapy involving the use of oligonucleotide compounds together with a GCS. This is contemplated to be able to reduce GCS consumption, and thereby reduce the cost, side-effects and risks associated with the said GCS therapy. Consequently, in this embodiment, said compound is administered together with a GCS.

A skilled person is well aware of the fact that there are several approaches to the treatment of inflammatory diseases, infectious diseases, neurodegenerative diseases, autoimmune diseases and cancer. Naturally new approaches are constantly being developed, and it is conceived that the oligonucleotides, their use and methods of treatment according to the present invention, will find utility also in combination with future treatments. The inventors presently believe that the inventive oligonucleotides, their use and methods of treatment would be useful as a stand-alone therapy for inflammatory and/or autoimmune diseases. It cannot however be excluded that the inventive oligonucleotides will have utility in combination with existing or future treatment therapies for these diseases.

The oligonucleotide is administered in a therapeutically effective amount. The definition of a "therapeutically effective dose" or "therapeutically effective amount" is dependent on the disease and treatment setting, a "therapeutically effective amount" being a dose which alone or in combination with other treatments results in a measurable improvement of the patient's condition. A skilled person can determine a therapeutically effective amount either empirically, or based on laboratory experiments, performed without undue burden. The treating physician can also determine a suitable amount, based on his/her experience and considering the nature and severity of the disease, as well as the patient's condition.

Another embodiment is the administration of the oligonucleotide in two or three or more separate amounts, separated in time by about 12 hours, about 24 hours, about 48 hours, about one week and about 4 weeks. Another embodiment is the administration of the oligonucleotide prior, in parallel or after the administration of a combination therapy.

The embodiments of the invention have many advantages. So far, the administration of an oligonucleotide in the amounts defined by the inventors has not elicited any noticeable side-effects. Further, the mucosal administration is easy, fast, and painless, and surprisingly results in a systemic effect. It is held that this effect, either alone, or in combination with existing and future treatments, offers a promising approach to fight the diseases of interest.

In another aspect of the invention, the oligonucleotides of the invention are useful for steroid re-sensitization.

In another aspect of the invention, the oligonucleotides of the invention are useful to influence the properties and behaviour of polymorphonuclear cells, in particular the recruitment and/or migration of polymorphonuclear cells to a site of inflammation, and that they through this mechanism have utility in the prevention, treatment and/or alleviation of various diseases such as ischemia.

### EXAMPLES

### Example 1: Relative structural composition of CD spectra of oligonucleotides of the invention

The fits of the CD spectra of eight reference oligonucleotides (Table 1 and Figure 1) overlaid on the measured CD spectra of selected oligonucleotides of the invention are presented in Figure 2. The tertiary, structural properties of the reference oligonucleotideas were analyzed by circular dichroism (CD) measurement and the resulting traces are presented in Figure 1. Prior to the CD measurements the oligonucleotide samples were annealed by heating to 90°C and then slow cooled in water bath to room temperature (20°C) over a period of 8 h. Selected oligonucleotides were annealed by heating to 90°C and then snap-cooled in ice-cold water bath at 4°C and then brought to room temperature. CD measurements in UV range were conducted on a Jasco J-720 spectropolarimeter (Jasco Corp., Tokyo, Japan). For the measurement 300 µl of the samples was loaded into a quartz cuvette with 0.1 cm path length (total volume 400 µl). The CD spectra of the oligonucleotide samples used for the calculation were collected at 20 µM concentration of the oligonucleotide. The CD measurements were conducted at the rate of 100 nm/min with 4 sec response time. Seven sequential spectra were taken for each sample with the final spectrum being the average of the 7 consecutive measurements. CD spectra of oligonucleotide samples were measured in 1 X PBS buffer (10mM phosphate buffer (pH 7.4) with 140mM NaCl and 27mM KCl). The blank spectrum of 1 X PBS was subtracted from the CD spectra of the samples solutions.

CD spectra were collected in the range from 190 to 350 nm at 25°C for all samples. The temperature in the sample holder was controlled and kept constant (±0.1°C) with the help of a Peltier element (PTC-348 WI). Data collection and evaluation were carried out by the software supplied with the instrument. The resulting spectra were normalized to the concentration of the oligonucleotide and zeroed at 320 nm.

For better visualization and analysis of the tertiary structure of oligonucleotides of the invention, a custom-built computer program was used to calculate the relative composition of the CD spectra. The program was written in MatLab (Mathworks Corp.). The algorithm is based on the assumption that the samples spectra are the linear combinations of the references spectra (Figure 1). The fitting of the experimental data were performed following Levenberg-Marquart non-linear least-squares algorithm. Only the data collected at high tension signal below approx. 600 V were considered reliable. Only the spectra measured in wavelength window from 200 to 350 nm have been included in the calculations of the relative structural composition of oligonucleotide samples.

### Example 2: Method for determination of oligonucleotide-stimulated cytokine production profile of healthy individuals represented by cultured peripheral blood mononuclear cells (PBMC) PBMC isolation, stimulation and cultivation

Buffy coats from healthy blood donors were obtained from the Karolinska University blood bank and used for preparation of peripheral blood mononuclear cells (PBMC). PBMC were isolated by density centrifugation on Ficoll Paque (Amersham Biosciences AB, Uppsala, Sweden). The cells were then further washed in PBS, and the viability and the cell number were determined by counting the cells in Trypan blue (Sigma Aldrich, Stockholm, Sweden). Thereafter the cells were re-suspended in complete cell medium consisting of RPMI 1640 (Sigma Aldrich) supplemented with 10 % heat inactivated fetal calf serum (FCS, Invitrogen), 2 mM L-glutamine, 100 U/mL penicillin, 100 µg/mL streptomycin, 10 mM HEPES (Sigma Aldrich) and 5 µg/mL gentamicin (Invitrogen). The PBMC were cultured in 48-well culture tissue plates (Becton Dickinson, Franklin Lakes, NJ) at a concentration of 5 x 10⁶ cells/mL with 10 µM of oligonucleotides of the invention, with medium alone as a negative control in a total volume of 400 µl/well. The cells were incubated for 48 h at 37 °C in a humidified cell culture incubator (Thermo Scientific, Waltham, MA) with 5% CO₂, in air, after which the culture supernatants were collected and frozen at -20°C for later cytokine analysis.

### Cytometric bead array measurements and data analysis

Culture supernatants from PBMC stimulated with oligonucleotides of the invention were analyzed for the presence of the cytokines IFN-γ, IL-6, IL-10 and IP-10 using cytometric bead array flex kit (Becton Dickinson) according to the manufacturer's instructions on a FACSArray flow cytometer (Becton Dickinson). The data were analyzed using FCAP Array software (Becton Dickinson).

### ELISA and data analysis

Culture supernatants from PBMC stimulated with oligonucleotides of the invention were analyzed for the presence of IFN-α using human IFN-α Multi-subtype ELISA kit (PBL, Biomedical Laboratories, NJ, USA) and IFN-β was detected with human IFN-β ELISA kit (Fujirebio INC. Tokyo, Japan) according to the manufacturer's instructions. The absorbance was measured on a microplate reader (Tecan, Männedorf, Switzerland) and the data were analyzed using Magellan software (Tecan). One set of results are shown in Table 4, which shows Cytometric Bead Array (CBA) mean data (IL6, IL10, IFN-γ, IP-10) from four healthy individuals and ELISA mean data (IFN-α, IFN-β) from six healthy individuals on cytokines and interferons production of PBMC cultured in presence of selected oligonucleotides of the invention.

**Table 4. Cytokine release induced by oligonucleotides with at least 40% tetramer/telomere form and controls**

| SEQ ID No | IDX-NO | IL6 | IL10 | **IFN-α** | **IFN-β** | **IFN-γ** | **IP-10** |
|---|---|---|---|---|---|---|---|
| 59 | 0465 | 2 | 1 | - | | - | - |
| 91 | 0445 | - | - | - | | - | - |
| 92 | 0455 | - | - | - | | - | 5 |
| 93 | 0460 | 4 | - | - | | - | 5 |
| 94 | 9134 | - | - | - | | - | 5 |
| 7 | 9011 | - | - | **1** | - | - | 5 |
| 81 | 9054 | 3 | 2 | **5** | **5** | 1 | 5 |
| 82 | 0440 | | | | | | |
| 83 | 9059 | 5 | 4 | **5** | **5** | 1 | 5 |
| 80 | 9133 | 3 | 3 | **3** | | - | 5 |
| 84 | 9004 | 3 | 2 | **5** | **3** | 1 | |
| 85 | 9005 | 2 | 2 | **5** | **5** | 1 | |
| 60 | 9008 | 2 | 3 | **5** | **1** | 1 | |
| 61 | 9018 | 2 | 2 | **5** | **4** | 1 | |
| 62 | 9019 | 1 | 2 | **5** | **2** | - | |
| 63 | 9020 | 1 | 1 | **5** | **4** | - | |
| 64 | 9021 | 2 | 2 | **5** | **3** | - | |
| 65 | 9023 | 2 | 3 | **5** | - | - | |
| 66 | 9026 | 3 | 3 | **5** | **3** | - | |
| 67 | 9027 | 3 | 3 | **5** | **2** | - | |
| 86 | 9030 | 4 | 3 | **5** | **5** | 1 | |
| 68 | 9039 | 2 | 2 | **4** | - | 1 | |
| 69 | 9040 | 2 | 2 | **5** | **1** | 1 | |
| 70 | 9041 | 4 | 3 | **5** | **1** | 1 | |
| 71 | 9042 | 3 | 3 | **5** | **1** | - | |
| 72 | 9047 | 2 | 2 | **5** | **2** | 1 | |
| 73 | 9048 | 2 | 2 | **5** | **4** | - | |
| 74 | 9049 | 3 | 4 | **5** | - | 1 | |
| 75 | 9050 | 2 | 3 | **5** | **2** | 1 | |
| 76 | 9055 | 4 | 3 | **5** | **5** | 2 | |
| 77 | 9057 | 3 | 3 | **5** | **5** | 1 | |
| 87 | 9060 | 3 | 2 | **5** | **5** | 1 | |
| 78 | 9067 | 4 | 2 | **5** | **1** | - | |
| 79 | 9068 | 1 | 1 | **5** | **2** | - | |
| 88 | 9045 | 5 | 4 | **5** | **5** | 2 | |
| 89 | 9053 | 5 | 3 | **5** | **1** | 1 | |
| 90 | 9058 | 5 | 4 | **5** | **5** | 1 | |

The mean concentration (pg/ml) of IFN-α, IFN-β and IP-10 is scored as follows;
(-) <125; 125≥(1)<250; 250≥(2)<500; 500≥(3)<750; 750≥(4)<1000; and 1000≥(5).

The mean concentration (pg/ml) of IL6, IL10 and IFN-γ is scored as follows;
(-)<65; 65≥(1)<125; 125≥(2)<250; 250≥(3)<500; 500≥(4)<750; and 750≥(5).

### Example 3: Relative tertiary composition of the CD spectra of selected oligonucleotides of the invention together with cytokine/interferon data.

The relative composition of tertiary structure of representative oligonucleotides of the invention, compared with biological data, are shown in Table 5.

**Table 5. Tetramer/telomere form contribution linked dominantly with IFN induction**

| SEQ ID NO | IDX-No | **% contribution of REF #1 (tetramer/ telomere)** | **% contribution of REF #2 (dimer/ fragile X)** | **% contribution of REF #5 (dimer/non-G-quadruplex)** | **% contribu tion of REF #7 (random )** | **% contributio n of REF #8 (monomer/ thrombin)** | IL | | **IFN** | | | IP-10 |
|---|---|---|---|---|---|---|---|---|---|---|---|---|
| | | | | | | | 6 | 10 | α | β | γ | |
| 7 | 9011 | 0 0 | 0 0 | 0 0 | 100 0 | 0 0 | - | - | - | - | - | + |
| 91 | 0445 | 15 3 | 0 0 | 0 0 | 49 1 | 35 6 | - | - | - | | - | - |
| 59 | 0465 | 22 2 | 35 4 | 28 0 | 5 2 | 5 0 | (+) | - | - | | - | - |
| 94 | 9134 | 25 4 | 41 7 | 6 0 | 22 7 | 4 1 | - | - | - | | - | + |
| 81 | 9054 | 61 0 | 0 0 | 14 8 | 17 5 | 7 7 | (+) | (+) | + | + | - | + |
| 83 | 9059 | 49 | 0 0 | 0 0 | 43 2 | 7 8 | + | + | + | + | - | + |
| 80 | 9133 | 44 3 | 0 0 | 10 1 | 43 8 | 1 8 | (+) | (+) | (+) | | - | + |

The mean concentration (pg/ml) of IFN-α, IFN-β and IP-10 is scored as follows; - < 250; 250 ≥ (+) < 750; 750 ≥ +.

The mean concentration (pg/ml) of IL6, IL10 and IFN-γ is scored as follows; - <125; 125 ≥ (+) < 500; 500 ≥ +.

The data in Table 5 shows that representative oligonucleotides that are present in at least 40% in tetramer/telomere form (related to Ref #1, SEQ ID NO 1) are capable of increasing levels of interferons. The data (IL-6, IL-10, IFN-α, IFN-β and IFN-γ are obtained in accordance with the methods set out in Example 1. The oligonucleotides of SEQ ID NO 7, SEQ ID NO 59, SEQ ID NO 91 and SEQ ID NO 94 represent control oligonucleotides, in that these oligonucleotides are not present in tetramer/telomere form of above 40% and, thus, according to the invention, are not capable of increasing levels of interferons.

## Claims

1. An isolated oligonucleotide which adopts, in aqueous solution, a tertiary structure of at least 40% of tetramer/telomere form, said oligonucleotide being capable of modulating the immune system in a mammalian subject upon administration to said subject.

2. The oligonucleotide of claim 1, which adopts, in aqueous solution, a tertiary structure of at least 60%, preferably 80%, more preferably 90% of tetramer/telomere form.

3. The oligonucleotide of claim 1 or claim 2, wherein said modulation of the immune system represents an increase in levels of cytokines.

4. The oligonucleotide of claim 3, wherein said cytokine represents interferon-α, interferon-β and/or interferon-γ.

5. The oligonucleotide according to any one of claims 1 to 4, selected from the group consisting of SEQ ID NOs 24 - 43, SEQ ID NOs 60 - 77 and SEQ ID NOs 79 - 80.

6. The oligonucleotide according to any one of claims 1 to 4, selected from the group consisting of SEQ ID NOs 60 - 77 and SEQ ID NOs 79 - 80.

7. The oligonucleotide according to any one of claims 1 to 4, wherein said oligonucleotide is selected from the group consisting of SEQ ID NO 76, SEQ ID NO 77 and SEQ ID NO 80.

8. The oligonucleotide according to any one of claims 1 to 7, which comprises at least one nucleotide that has a phosphate backbone modification.

9. The oligonucleotide of claim 8, wherein said phosphate backbone modification is a phosphorothioate or phosphorodithioate modification.

10. The oligonucleotide according to any one of claims 1 to 9, which comprises of about 5 to about 120 nucleotides, preferably of about 12 to about 30 nucleotides.

11. The oligonucleotide according to any one of claims 1 to 10, for use in therapy.

12. The oligonucleotide according to any one of claims 1 to 10, for use in treating a disease where increasing the levels of cytokines are beneficial for said treatment.

13. The oligonucleotide according to any one of claims 1 to 10, for use in the treatment of diseases selected from infectious diseases, inflammatory diseases, neurodegenerative diseases, autoimmune diseases and cancer.

14. The oligonucleotide according to any one of claims 1 to 10, for use in the treatment of diseases selected from inflammatory bowel disease, hairy cell leukemia, haematological malignancy, multiple sclerosis, hepatitis B, hepatitis C, chronic hepatitis, cirrhosis, chronic granulomatosis disease and severe malignant osteopetrosis.

15. Use of an oligonucleotide according to any one of claims 11 to 14, which use further comprises one or more additional agents effective in treating infectious diseases, inflammatory diseases, neurodegenerative diseases, autoimmune diseases or cancer.

16. A pharmaceutical composition comprising an oligonucleotide according to any one of claims 1 to 10, together with pharmaceutically acceptable adjuvants, diluents or carriers.
